# EUROPEAN PATENT APPLICATION

(11) **EP 3 420 907 A2**
(43) Date of publication of application: **02.01.2019**
(21) Application number: 18179522.0
(22) Date of filing: 25.06.2018
(51) Int. Cl.: A61B 6/00

(54) **VARIABLE DISTANCE IMAGING**

(30) Priority: 30.06.2017 US 201715638499
(71) Applicant: General Electric Company, Schenectady, NY 12345 (US)
(72) Inventor: MULLER, Serge Louis Wilfrid, 78530 FR BUC (FR); WEAR, James A., Madison, WI 53718 (US); MARKWARDT, Paul Eugene, Madison, WI 53718 (US); PAYNE, Randall Kenneth, Madison, WI 53718 (US)
(74) Representative: Bedford, Grant Richard

(57) **Abstract**

A system 10 for imaging includes a gantry 12 movable relative to a subject. A source 14 is configured to emit radiation during an imaging procedure. A detector 18 is configured to receive attenuated radiation from the source 14 during an imaging procedure, at least one of the source 14 and the detector 18 movably secured to the gantry 12 by an adjustable joint 50,54. An imaging controller 28 is operably connected to at least the gantry 12 and to the adjustable joint 50,54, wherein the gantry controller 28 receives a priori patient information and imaging system geometry information, the imaging controller determines an imaging geometry and operates the gantry 12 and the adjustable joint 50,54 to vary a source 14 to image-receptor distance (SID) according to the imaging geometry.

## Description

The present disclosure is related generally to the field of medical diagnostic imaging. More specifically the present disclosure is directed to systems and methods of medical imaging particularly related to bone densitometry.

In medical x-ray imaging, for example, bone densitometry systems, an x-ray source and an x-ray detector are generally mounted on opposing ends of a substantially C-shaped gantry. A scanning radiographic technique, such as typically employed with densitometry, uses a narrowly collimated beam of radiation formed into, for example a fan beam. The emitted fan beam of radiation, typically x-rays, are incident on and detectable by the x-ray detector, although other configurations of x-ray imaging systems are known. This typically uses a smaller array for the x-ray detector, and the x-ray source and the x-ray detector are moved relative to the patient. In embodiments, this enables scanning or collection of data from a broad area of the patient, including the entire patient, as compared to other conventional radiography techniques. The source and the detector are positioned such that when an object (e.g., part of a human body) is interposed there between and is irradiated with x-rays, the detector produces data representative of characteristics of the interposed object.

In the particular application of densitometry, when two (or more) energies of x-rays are used, bone and tissue information can be acquired due to the differences in the absorption of the x-rays of different energies. Measurements of the x-ray absorption by an object at two different x-ray energies can reveal information about the composition of that object as decomposed into two selected basis materials. In the medical area, the selected basis materials are frequently bone and soft tissue. The ability to distinguish bone from surrounding soft tissue allows x-ray images to yield quantitative information about in vivo bone density for the diagnosis of osteoporosis and other bone disease.

At least some known dual-energy imaging systems include detector elements that are fabricated using a Cadmium Telluride (CdTe) semiconductor having Schottky anode and cathode contacts. Under the influence of an applied biasing voltage, the semiconductor generates a current proportional to the energy of each x-ray absorbed by the semiconductor. The slight increases in the semiconductor current due to the x-rays are translated in to digital signals that are used to generate an image.

An exemplary embodiment of an imaging system includes a movable table configured to support a patient to be imaged. A gantry is movable about the movable table. The gantry includes at least one adjustable joint. A source is configured to emit radiation during an imaging procedure. A detector is configured to receive attenuated radiation from the source during the imaging procedure. At least one of the source and the detector are movable relative to the other by the at least one adjustable joint of the gantry. An imaging controller is operably connected to at least the movable table, the gantry, and to the at least one adjustable joint. The imaging controller operates at least one of the movable table, the gantry, and the at least one adjustable joint to change relative positions between the source, the detector, and the table.

In exemplary embodiments of the imaging system, the imaging controller further receives imaging procedure information and imaging system geometry information. The imaging controller determines an imaging geometry and operates the gantry and the at least one adjustable joint to vary a source to image-receptor distance (SID) according to the imaging geometry. In a further exemplary embodiment, an adjustable collimator is associated with the source. The adjustable collimator is operable by the imaging controller to shape a beam of radiation emitted from the source based upon the SID of the imaging geometry.

In exemplary embodiments of the imaging system, the source is an x-ray emitter and the detector is an x-ray detector. The imaging controller acquires medical images in the form of x-ray images during an imaging procedure. An emitter joint movably connects the x-ray emitter to the gantry and a detector joint movably connects the x-ray detector to the gantry. The imaging controller operates the emitter joint and the detector joint in coordination to adjust at least one of the SID, a source to object distance (SOD), and an object to image-receptor distance (OID).

In exemplary embodiments of the imaging system, the imaging controller receives imaging procedure information and imaging system geometry information. The imaging controller determines an imaging geometry that includes a source trajectory and a detector trajectory. The imaging controller operates the gantry during the imaging procedure according to the source trajectory and the detector trajectory. In a further exemplary embodiment, the imaging controller operates at least one of the emitter joint and the detector joint during the imaging procedure to provide a source trajectory and a detector trajectory that results in a varying SID during the imaging procedure.

The present invention will be better understood from reading the following description of non-limiting embodiments, with reference to the attached drawings, wherein below:
FIG. 1 is a block schematic diagram of an exemplary imaging system.
FIG. 2 is a schematic diagram of an exemplary densitometry system.
FIG. 3 is a schematic diagram of a C-arm assembly according to an embodiment of the invention.
FIG. 4 diagrammatically depict source and detector trajectories of an imaging geometry with respect to a subject.
FIG. 5 exemplarily depicts two orientations of the source and detector.

The following description relates to various embodiments of medical imaging systems. In particular, methods and systems are provided for use as a single energy x-ray absorptiometry (SXA) system, as is exemplarily used to measure breast density or a dual-energy x-ray absorptiometry (DXA) used to measure bone mineral density. Examples of densitometry are used herein although it will be recognized that in other embodiments, other modalities of radiography and/or medical imaging may be employed. For example, these may include, but are not limited to: tomosynthesis, MRI, PET, SPECT, C-arm angiography, mammography, ultrasound, and so forth. The present discussion of densitometry is provided as an example of one suitable application.

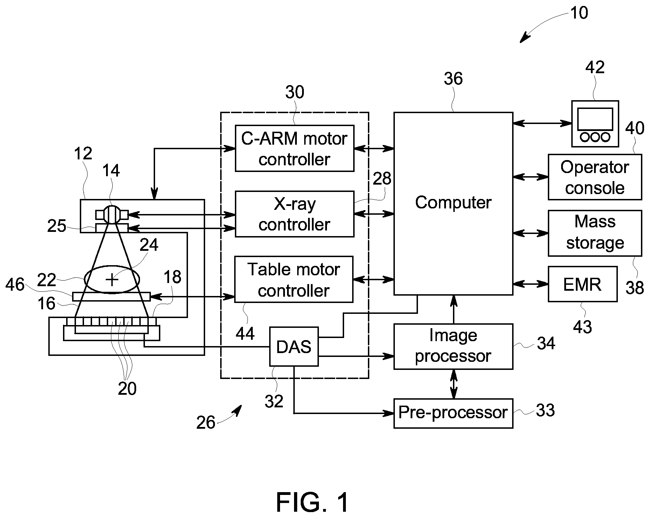

In exemplary embodiments, as variously depicted in Figs. 1-3, the densitometry system 10 maybe configured to include a substantially C shaped or semi-circular gantry, or C-arm 12. The C-arm 12 movably supports a source 14 and a detector 18 mounted opposite to each other on opposed ends. The patient is disposed between the source 14 and the detector 18.

It will be recognized that in other imaging systems within the present disclosure, one of the source or detector may remain in a fixed position while the other of the source or detector is movable with respect to the patient. In still other exemplary embodiments as disclosed herein, the table, which is configured to support the patient, is further movable to achieve a desired image acquisition. During an acquisition of image data, the C-arm 12 is movable to change a position and/or orientation of the source 14 and/or detector 18 relative to the patient. In an exemplary embodiment, the C-arm 12 may move the source 14 and the detector 18 in a transverse scanning path, a progressive overlapping scanning path, or a zig-zag (e.g. raster) scanning path. It will be recognized that other forms of image data acquisition may utilize other forms of scanning paths, which may include, but are not limited to rotation or tilt of the C-arm 12.

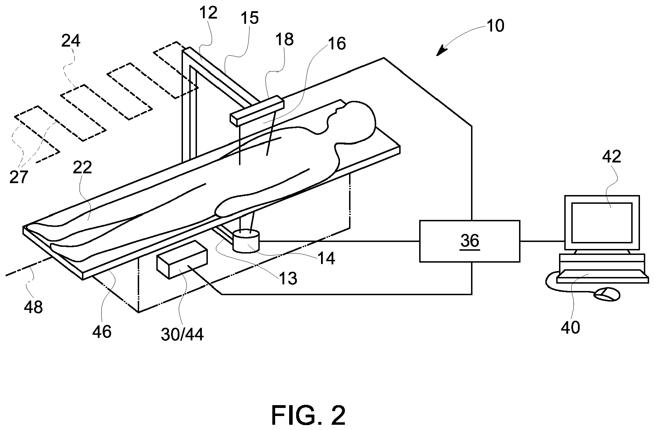

Referring to Figures 1 and 2, an exemplary embodiment of the system 10 is constructed to measure at least an area of a bone, a length of bone, a bone mineral content (BMC), a bone mineral density (BMD), or a tissue thickness or density. The BMD is calculated by dividing the BMC by the area of a bone. During operation, an x-ray beam with broadband energy levels is utilized to scan an object, for example, to scan a human patient to image the bones of the patient. The acquired images of the bones are used to diagnose a medical condition, for example osteoporosis. The images may be generated in part from determined bone density information acquired during a dual-energy x-ray scan. As described in further detail herein, the positions of the source 14, detector 18, and/or table can be adjusted to achieve further desired imaging purposes, including but not limited to magnification, increasing image resolution, or spatial resolution.

The imaging system 10 is shown as including a gantry 12. For exemplary purposes, the imaging system 10 may be described as a dual-energy x-ray absorptiometry (DXA) system, although it will be recognized that a variety of other systems may also be implemented in a similar manner. Gantry 12 includes an x-ray source 14 that projects a beam of x-rays 16 toward detector array 18. The gantry 12 exemplarily includes a lower end 13 that is positioned below a subject 22, such as a patient, and an upper end 15 that is positioned above the subject 22. The x-rays pass through the subject 22 to generate attenuated x-rays. As depicted in Fig. 1, the x-ray source 14 may be secured to the upper end 15 and the x-ray detector 18 secured to the lower end 13. As depicted in Fig. 2, the detector 18 maybe secured to the upper end 15 and the x-ray source 14 maybe secured to the lower end 13. Each detector element 20 is exemplarily, but not limited to a cadmium telluride (CdTe) detector element, which produces an electrical signal that represents an intensity of the attenuated x-rays. During a scan to acquire image data, gantry 12 and/or components mounted on gantry 12 are exemplarily movable relative to the subject 22 and/or a table 46.

Movement of the gantry 12 and an operation of x-ray source 14 are governed by an imaging controller 26 of imaging system 10. Imaging controller 26 includes an x-ray controller 28 that provides power and timing signals to x-ray source 14. The x-ray controller 28 may further provide operational and/or control signals to the adjustable collimator 25 to shape the beam of x-rays from the source 14 in accordance with the imaging procedure to be performed. In embodiments, the x-ray beam may be shaped (collimated) as a fan beam. In an exemplary embodiment, the fan beam 16 may be a narrow fan beam such as to limit the divergence between x-rays in the beam, which has been shown to improve parallax and image overlap blurring.

The imaging controller 26 further includes a gantry motor controller 30 that controls a motion, speed, and position of gantry 12. In some embodiments, gantry motor controller 30 may control a tilt angle of gantry 12. The gantry motor controller 30 may further operate to control a movable joint 50 between the detector 18 and the gantry 12. The gantry motor controller 30 may further operate to control a movable joint 54 exemplarily between the source 14 and the gantry 12. The table motor controller 44 is operably connected to the table 46 through a table motor 70. The table motor 70 is operable, under control signals from the table motor controller 44, to translate, rotate, and/or tilt the table 46 in a plurality of degrees of freedom of movement. In an embodiment, the table motor 70 is operable to move the table 46 in three degrees of freedom, (e.g. horizontal, vertical, and depth translation) while in another embodiment, rotational degrees of freedom of movement (e.g. pitch, yaw, and roll) may be available. It will be recognized that the table motor 70 may include one or more mechanical or electromechanical systems to carry out these movements of the table 46, including but not limited to tack and opinion, screw, or chain driven actuators.

The x-ray source 14 and the x-ray detector 18 may be moved in a raster pattern 24 so as to trace a series of transverse scans 27 of the subject 22 during which dual energy x-ray data is collected by the x-ray detector 18. The transverse scanning procedure generates either a single image or quantitative data set, form a plurality of scan images acquired across a patient, wherein the x-ray source 22 and the detector 26 are either longitudinally aligned with the superior-inferior axis of the patient or transversely from the patient's left to right. Scanning a patient using a transverse motion facilitates minimizing the time between acquisitions of adjacent scan images because the transverse direction across the patient is shorter than the longitudinal direction across the patient. Thus transverse scanning can reduce the severity of patient motion artifacts between scan images allowing the images to be more accurately merged.

The transverse scanning motion is produced by coordination between the motion control of the gantry 12, x-ray source 14, and the x-ray detector 18 by the gantry motor controller 30 as well as control of the table 46 by the table motor controller 44 which operates the table 46 through the table motor 70. During operation, the x-ray source 14 produces a fan beam 16 having a plane that is exemplarily parallel to the longitudinal axis 48. Optionally, the fan beam 16 may have a plane that is perpendicular to the longitudinal axis 48. The raster pattern 24 is adjusted such that there is some overlap (e.g., an overlap of 10%) between successive scan lines of the fan beam 16.

A data acquisition system (DAS) 32 exemplarily in the imaging controller 26, samples and digitizes the data from detector elements 20 and converts the data to sampled and digitized data for subsequent processing. In some embodiments, DAS 32 may be positioned adjacent to detector array 18 on gantry 12. Pre-processor 33 receives the sampled and digitized data from DAS 32 to pre-process the sampled and digitized data. In one embodiment, pre-processing includes, but is not limited to, an offset correction, a primary speed correction, a reference channel correction, an air-calibration, and/or applying a negative logarithmic operation. As used herein, the term processor is not limited to just those integrated circuits referred to in the art as a processor, but broadly refers to a controller, a microcontroller, a microcomputer, a programmable logic controller, an application specific integrated circuit, and any other programmable circuit, and these terms are used interchangeably herein. Pre-processor 33 pre-processes the sampled and digitized data to generate pre-processed data.

An image processor 34 receives the pre-processed data from pre-processor 33 and performs image analysis, including that of densitometry and/or absorptiometry through one or more image processing operations. The acquired bone and tissue information, for example, image and density information may be processed and displayed in real time though operations to the image processor 34 and/or the computer 36. The computer 36 exemplarily operates to store the reconstructed image in a mass storage device 38, where the mass storage device 38 may include, as non-limiting examples, a hard disk drive, a floppy disk drive, a compact disk-read/write (CD-R/W) drive, a Digital Versatile Disc (DVD) drive, a flash drive, and/or a solid-state storage device. As used herein, the term computer is not limited to just those integrated circuits referred to in the art as a computer, but broadly refers to a processor, a microcontroller, a microcomputer, a programmable logic controller, an application specific integrated circuit, and any other programmable circuit, and these terms are used interchangeably herein. It will be recognized that any one or more of the processors and/or controllers as described herein may be performed by, or in conjunction with the computer 36, for example through the execution of computer readable code stored upon a computer readable medium accessible and executable by the computer 36.

Computer 36 also receives commands and scanning parameters from a user, such as an operator, via a console 40 that includes a user interface device, such as a keyboard, mouse, voice-activated controller, touchscreen or any other suitable input apparatus. An associated display 42 allows a user, such as an operator, to observe the image and densitometry data from computer 36. The commands and scanning parameters are used by computer 36 to provide control signals and information the imaging controller 26, including the DAS 32, x-ray controller 28, and gantry motor controller 30. In addition, computer 36 may operate a table motor controller 44 exemplarily of the imaging controller 26 which controls a movable subject support, which is exemplarily a motorized table 46, to position subject 22 within gantry 12. Particularly, table motor controller 44 adjusts table 46 to move portions of subject 22.

During operation, the system 10 is configured to operate in either a dual energy x-ray mode or a single energy x-ray mode. In the single energy mode, the x-ray source 14emits x-rays at a narrow band of energies of a few keV and in the diagnostic imaging range of approximately 20-150 keV. In the dual-energy mode, the x-ray source 14 emits radiation at two or more bands of energy emitted simultaneously or in rapid succession. The x-ray source 14 may also be configured to emit a single broadband energy of more than a few keV over the diagnostic imaging range. The system 10 may be switched between the dual energy mode and the single energy mode by increasing or decreasing the x-ray source 14 voltage and/or current. The system 10 may also be switched between the dual energy mode and the single energy mode by removing or adding a Kedge filter. It should be noted that the x-ray source 14 may emit x-rays at different energies or ranges of energies.

The x-ray source 14 may be configured to output a fan beam 16 of x-rays. The x-ray source 14 may also be configured to output a pencil beam of x-rays (not shown), a cone beam of x-rays, or other configurations. In some embodiments, the computer 36 controls the system 10 to operate in the single energy mode or dual-energy mode to determine the bone or tissue information of at least some of the scanned body. The single energy mode generally enables higher resolution images to be generated. The acquired images may then be used to measure, for example, bone density or other bone and tissue characteristics or content. As discussed above, the dual-energy x-ray scan may be a rectilinear scan of the entire patient body, which may be performed in a transverse-type scanning sequence as described above. During the dual-energy x-ray scan an image of the entire body of the patient may be acquired, which includes image information relating to the bones and tissue in the body. The full body or total body scan of the entire body may be performed as a single scanning operation, which may be a low dose mode scan. In some embodiments, instead of a full body or total body scan, individual rectangular regions of the body may be performed, which may be single sweep scans. Once the scan of the patient, or a portion thereof, is completed, the dual energy signals provided by the detector 18 are deconstructed into images of two basis materials, such as bone and soft tissue. The high and low energy signals can also be combined to provide a single energy mode having superior signal to noise ratio for imaging purposes.

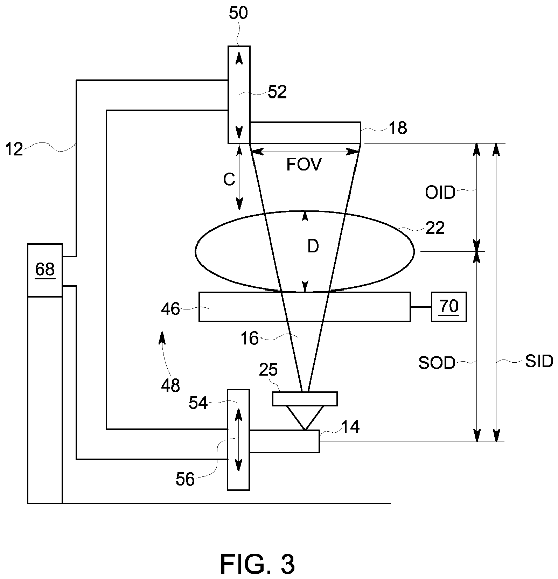

As described in further detail herein with reference to Fig. 3, the gantry motor controller 30, for example under operation from the computer 36, may further operate to control a movable joint 50 between the detector 18 and gantry 12. The movable joint 50 is operated by the gantry motor controller 30 to move the position of the detector exemplarily towards and away from a center point of the gantry 12 along line 52. Similarly, the gantry motor controller 30 may operate a movable joint 54 between the source 14 and the gantry 12. The movable joint 52 is operated by the gantry motor controller 30 to move the position of the source 14 exemplarily towards and away from a center point of the gantry 12 along line 56. The movable joints 50, 54 may be any of a variety of mechanical movable joints, including, but not limited to rack-and-pinion, screw, or chain driven actuators. Operation of the movable joints 50, 54 control the SID, SOD, and OID and described in further detail herein.

The gantry motor controller 30 is further operatively connected to a motorized gantry joint 68. The motorized gantry joint 68 is exemplarily operable to move the gantry C-arm 12 in coordinate space. For example, the motorized gantry joint 8 maybe operable to move the C-arm 12 in between one and three dimensions. In one embodiment, the motorized gantry joint 68 is operable to move the C-arm in a horizontal and a depth dimension as well. As is known, the motorized gantry joint 68 is operable, under the control of the gantry motor controller 30, to rotate the C-arm 12 about an axis. In the exemplary embodiment depicted, the motorized gantry joint 68 is operable, under the control of the gantry motor controller 30, to rotate the C-arm about at least two axes.

It will be recognized that in still further embodiments, adjustable SID, SOD, and OID may exemplarily be provided by independently driving the source and the detector, for example, in a system without a C-arm physically connecting the source and the detector. In another exemplary embodiment, the at least one moveable joint may be provided on the gantry 12, e.g. c-arm, such movable joint being operable to adjust the relative position between the source 14 and the detector 18.

A field of view (FOV) of an imaging procedure is exemplarily dependent upon the relationship between the source, the detector, and the patient. The FOV and image quality may exemplarily be dependent, at least in part upon the relative positions of components within the imaging system. As exemplarily depicted in Figure 3, these include a distance between the source and the detector (SID), the distance between the source and the object as represented by the center of the ROI (SOD) and the distance between the detector and the center of the ROI (OID). To increase the FOV, one may decrease the OID and increase the SOD. However, the patient's body, or other object supporting the patient, the imaging system itself, and/or the arrangement of the imaging room may create further constraints on the positions of the source and detector. In embodiments as disclosed in further detail herein, improved imaging can be obtained by varying the positions of one or more of the source, the detector, and the table before and/or during an imaging procedure. The positions of the source, the detector, and the table can be varied in consideration of the ROI, the geometry of the imaging system, the size of the patient. These variations may be made inter-procedure or these variations may be made intra-procedure.

In an exemplary embodiment, the performance and robustness of a DEXA imaging system can be improved with refined control of the relative locations of the x-ray source 14, the x-ray detector 18, and the subject 22 (via manipulation of the table 46). Subjects vary greatly in size and shape. In an exemplary embodiment, the source 14 is typically located at a fixed relationship below the table 46. The detector 18, however, can be moved towards or away from the subject 22 to accommodate the size of the subject, particularly the subject's girth. In embodiments, where the subject is thinner, the detector 18 may exemplarily be moved closer to the subject 22. By reducing the OID, more x-ray flux is received per cell of the detector 18, improving image quality all other factors remaining constant. However, while normally the detector location as represented by the upper arm of the C-arm is fixed at a location to balance average subject size and desired image quality, in embodiments as described herein, the C-arm can be operated to increase the OID such that a larger patient may be accommodated between the detector 18 and the table 46. In other embodiments, the table 46 may be moved lower; however, without a corresponding shift in the position of the x-ray source as described herein, the SOD will be reduced, resulting in a reduced FOV. In the context of imaging a large patient, a reduced FOV may increase imaging time, expose the subject to greater x-radiation, or be counter to imaging procedure goals.

Related to that as described above, by moving the source closer to the patient, the SOD is reduced and while this reduces the FOV of the imaging procedure, the resolution of that imaging procedure is improved as the cells of the detector are spread across a smaller area of the patient being imaged. This may be particularly useful in embodiments wherein greater magnification of a smaller ROI of the patient is desired. In one example, this may be used to image particular joints for evaluation of bone degeneration.

The imaging controller exemplarily operates to determine a scanning pattern 24, which as described above, may be a raster pattern. The gantry 12, table 46, the x-ray source 14 and the x-ray detector 18 are exemplarily moved to follow the determined scanning pattern 24. The scanning pattern 24 may include a plurality of transverse scans 27, which may exemplarily depend upon a width of a fan beam projected by the x-ray source 14 and collimator 25. The scanning pattern 24 may further include one or more of the adjustments to the SID, SOD, or OID as described above, for example based upon inputs from the user to control the imaging geometry or the objectives of the procedure, for example to scan a particular anatomical portion of the patient.

In an exemplary embodiment, a contour of the patient may be followed, for example as the transverse scans 27 of the raster pattern 24 are performed, the position of the x-ray source 14 and/or the x-ray detector 18 may be moved relative to an envelope or a contour of the subject 22 on the table 46. In such an embodiment, each of the transverse scans may have an SID, SOD, an OID as determined relative to the dimensions of the patient cross section at that transverse section and/or the particular investigation of the imaging. In an exemplary embodiment, the imaging pattern 24 may include one or more adjustments to at least one of SID, SOD, or OID for one or more of the transverse scans 27 included therein.

In exemplary embodiments, patient contour information, either acquired by one or more scout images, stored patient size and/or shape data, patient height, weight, BMI, or other physical measurements can be used to determined adjustments of the source position, table position, detector position and/or position of the gantry 12. In an embodiment, a digital patient model may be created and/or already stored in the patient's EMR. In embodiments, the system 10 may include an imaging device, for example, but not limited to a digital camera that acquires one or more images of the patient, the images may be acquired from one or more positions and patient size/length/volume measurements obtained from these images. In other embodiments, an initial, low dose, or scout scan of the patient may be acquired from which patient measurements may be made. The patient contour may exemplarily be an envelope bounded by the surface of the table on one side and a depth/height (D in Fig. 3) above the table representing the patient. In embodiments, the contour may exemplarily be a predetermined distance or clearance height (C) above the highest portion of the patient. If more detailed models or measurements of the patient are available, the patient contour may similarly be adjusted to more accurately reflect anatomical portions of the patient relative to the table.

While the present descriptions have been made with respect to a system in which the x-ray source 14 is located below the subject 22 and the x-ray detector 18 is located above the subject, it will be recognized that similar embodiments may be implemented with the x-ray source 14 and x-ray detector 18 positions reversed.

In embodiments, the computer 36 may additionally comprise or operate all or part of the imaging controller 26, including, but not limited to the x-ray controller 28, gantry motor controller 30, DAS 32, pre-processor 33, image processor 34, and table motor controller 44. It will be recognized that these components may be implemented in one or more processors or controllers and perform the functions as described herein in coordination among such controllers or as modules or programs operating on a single computer or controller.

In an alternative embodiment, a high frequency electromagnetic energy projection source configured to project high frequency electromagnetic energy toward subject 22 may be used instead of x-ray source 14. A detector array disposed within a gantry and configured to detect the high frequency electromagnetic energy may also be used instead of detector array 18.

In one embodiment, the image processor 34 stores the reconstructed images in the mass storage device 38. Alternatively, the image processor 34 transmits the image data to the computer 36 for generating useful patient information for diagnosis and evaluation. In certain embodiments, the computer 36 transmits the image data and/or the patient information to a display 42 communicatively coupled to the computer 36 and/or the image processor 34. In some embodiments, patient information may be collected from an external source, possibly electronically, for example, as stored in an Electronic Medical Record (EMR) 43 and may also be entered by the operator of the machine.

In one embodiment, the display 42 allows the operator to evaluate the imaged anatomy. The display 42 may also allow the operator to select an ROI and/or request patient information, for example, via graphical user interface (GUI) for a subsequent scan or processing.

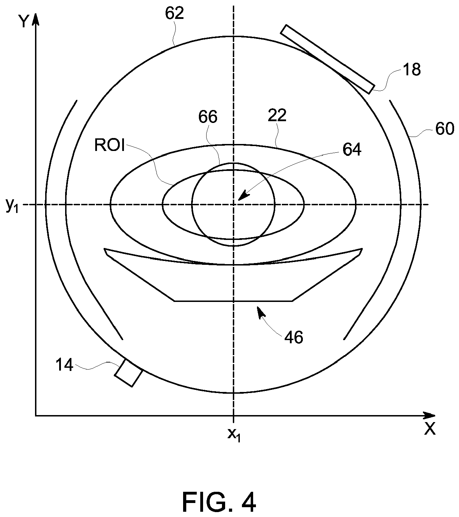

Figure 4 diagrammatically depicts an exemplary imaging geometry between a source trajectory 60 and a detector trajectory 62 with respect to a subject 22 as may be used in an embodiment as disclosed herein in the application of a CT imaging system. Figure 4 exemplarily represents the patient 22 positioned on the movable table 46. By operation of the gantry 12 as explained above, the x-ray source 14 is movable along a source trajectory 60 and a detector 18 is movable along a detector trajectory 62. The patient 22 is exemplarily located centrally to the source trajectory 60 and to the detector trajectory 62. The source trajectory 60 and the detector trajectory are exemplarily achieved by maintaining the positions of the source 14, the detector 18, and the table 46 while simultaneously rotating the source 14 and the detector 18, for example with the gantry (not depicted). This shows an exemplary embodiment where the SID, SOD, and OID all remain fixed throughout the imaging procedure.

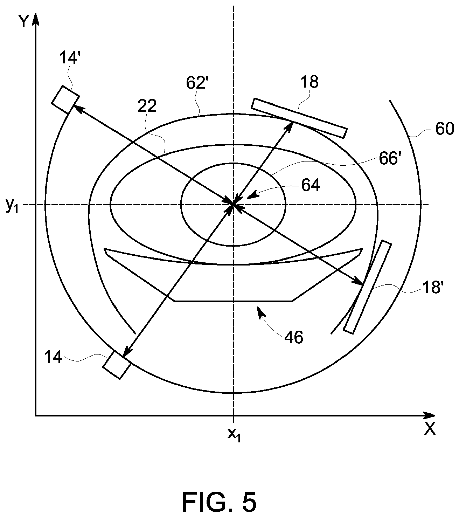

In Figure 5, an exemplary CT system as depicted in Fig. 4 is presented with the detector trajectory 62' modified, exemplarily to follow a contour of the moveable table 46 and the patient 22. In such an embodiment, the detector trajectory 62' may be achieved by changing the position of the detector 18 towards or away from an isocenter 64 of the subject 22, exemplarily about which the rotation axis of the gantry supporting the source 14 and the detector 18 is positioned. Movement of the position of the detector 18 toward and away from the isocenter 64 and/or gantry axis of rotation varies the SID and OID while providing a fixed SOD.

The C-arm gantry defines an axis of rotation about which the source and detector are rotatable. By positioning this axis of rotation at or near an object, and by rotating the source and detector about the object, or rotating the object about the source and detector, images of the object taken at a plurality of different orientations can be obtained. These images can be combined to generate a comprehensive three-dimensional image of the object, for example using methods of image reconstruction. Such acquisitions are usually called cone-beam computed tomography (CBCT) acquisitions.

CBCT capable systems typically provide a small field of view and thus can only 3D image a small portion of an object (e.g. patient) during a single scan. When imaging an off-center portion of an object, for example, a liver of a patient, the table upon which the patient rests during the scan is typically positioned such that the anatomy of interest coincides with the 3D field of view. However, it is possible that the detector and/or the source may collide with the patient because the patient is now positioned closer to the trajectories of the detector and/or the source. Moving the detector away from the center of the rotation reduces collision risk, but further reduces the diameter of any reconstructed three-dimensional image of the object. Currently, imaging system operators use a trial-and-error approach wherein the patient is repositioned so that no such collisions occur. In some instances, repositioning the patient may lead to the anatomy of interest lying outside of the imaging field of view. Reduced field of view or improper patient positioning can potentially lead to additional acquisition, resulting in increased x-ray dose, prolonged medical procedure and/or additional use of chemical injectable agent.

Typically, a subject to be imaged, such as a patient, is positioned within the imaging plane such that radiation generated by the source 14 passes through the subject and is detected by the detector 18. For cone beam CT (CBCT), the field of view (FOV) of the imaging system is small and centered on the isocenter. In some instances, the region of interest (ROI), which may exemplarily be a particular organ, organ system, or object to be imaged, may be off-center with respect to the subject, and so the subject should be positioned such that the FOV coincides with the ROI. In embodiments, an adjustable collimator 25 is positioned in association with the x-ray source 14. The adjustable collimator 25 operates to shape the beam of x-rays 16 emitted from the x-ray source 14 in connection with an imaging procedure.

As noted above, by varying one or more of SID, SOD, and OID, the trajectory of one or both of the x-ray source 14 and the detector 18 can be controlled to accommodate the size of the patient, while reducing or preventing any collision risk between either of the source and detector with the table and/or patient. Exemplarily referring to Figure 4, varying of the SID, SOD, and OID, along with achieving a change to the imaging isocenter may further be produced by adjusting the position of the table 46, either prior to an imaging procedure, or during an imaging procedure.

Figure 5 exemplarily depicts two orientations of the source 14 and detector 18. It will be recognized that in an exemplary embodiment, the gantry C-arm which physically connects and simultaneously moves the source 14 and detector 18 are not depicted. The source 14 and detector 18 are exemplarily shown at two positions along the respective source trajectory 60 and detector trajectory 62'. As can be seen in an examination of Figure 3B, at the positions of source 14' and detector 18' x-rays are emitted in the direction between the source 14' and the detector 18' across an SID comprised of an SOD and an OID. At the positions of source 14 and detector 18, the SID has been reduced due to the varying of the relative detected position along the detector trajectory 62' which moves the detector closer to the isocenter 64 of the patient (object). In the example depicted in Figure 3B, the SOD remains the same but the reduction in the OID results in an overall reduced SID at this point in the imaging procedure.

The adjustable nature of the imaging system as disclosed herein exemplarily provides an imaging system with robust imaging capabilities which are adaptable to various imaging procedures as well as patient sizes. By providing a gantry that can achieve variable SID, SOD, and OID on an interprocedure basis as well as an intraprocedure basis, embodiments of the system as disclosed herein can be effectively used to provide imaging procedures on both infants or children as well as bariatric adults. In previous imaging systems, interprocedure or intraprocedure adjustments to accommodate patient size were limited or not available. In exemplary embodiments this may position the source and detector close to the patient to achieve the goals of the imaging procedure while avoiding risk of collision with any of the components of the imaging system with the patient and/or table.

The description as provided herein has used the exemplary embodiment of DEXA. While other imaging techniques may also find similar benefits with the systems and methods as disclosed herein, various embodiments may produce the technical effects of the advantages as described in the present application. In an exemplary embodiment, it will be recognized that in general a reduced SID is associated with obtaining improved image quality and/or an equivalent imaging quality at a lower radiation dose and therefore in embodiments, the trajectory of the source and/or detector may be selected and/or operates minimizing radiation dose while still achieving a desired image quality of the selected image procedure.

In one exemplary embodiment, the system disclosed herein may be used to perform a high resolution imaging mode, for example for joint imaging. In the high resolution mode, the system is operated to reduce the SOD, for example by operating the movable joint 54 to position the source 14 closer to the center of the rotation axis of the gantry 12. In another embodiment, the table motor 70 may be operated to position the table, and the patient support by the table, closer to the source 14. In either event, with a reduced SOD, the object is magnified, providing the ability to capture increased resolution image.

In another exemplary embodiment, the SID may be reduced, for example, in the case of pediatric imaging wherein the patient is smaller enabling a smaller SID. By reducing SID, flux is increased at the detector elements level and special resolution can be maintained with a higher resolution detector e.g. a detector including four rows instead of a detector using two rows of detector elements.

In exemplary embodiment, the adjustable collimator 25 may be operated in connection with changes in the SID, such that the beam of x-ray 16 is shaped to conform to the two dimension field of view (FOV) as is exemplarily constrained by the SID and the size of the detector array 18.

In still further exemplary embodiments, while fan beam radiography has been used for exemplary proposes herein, it will be recognized that other shapes of x-ray beams may be used in various imaging procedures. For example, the adjustable collimator associated with the source 14 may be used to provide other beam shapes, including, but not limited to, cone-beams, rectilinear beams, narrow fan-beams, or wide fan-beams, although a person of ordinary skill in the art will recognize other beam shapes as may exemplarily also be provided in other embodiments while remixing within the scope of the present disclosure.

In the above description, certain terms have been used for brevity, clarity, and understanding. No unnecessary limitations are to be inferred therefrom beyond the requirement of the prior art because such terms are used for descriptive purposes and are intended to be broadly construed. The different systems and method steps described herein may be used alone or in combination with other systems and methods. It is to be expected that various equivalents, alternatives and modifications are possible within the scope of the appended claims.

The functional block diagrams, operational sequences, and flow diagrams provided in the Figures are representative of exemplary architectures, environments, and methodologies for performing novel aspects of the disclosure. While, for purposes of simplicity of explanation, the methodologies included herein may be in the form of a functional diagram, operational sequence, or flow diagram, and may be described as a series of acts, it is to be understood and appreciated that the methodologies are not limited by the order of acts, as some acts may, in accordance therewith, occur in a different order and/or concurrently with other acts from that shown and described herein. For example, those skilled in the art will understand and appreciate that a methodology can alternatively be represented as a series of interrelated states or events, such as in a state diagram. Moreover, not all acts illustrated in a methodology may be required for a novel implementation.

This written description uses examples to disclose the invention, including the preferred mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. A system for medical imaging:
   a gantry movable relative to a subject, the gantry comprising at least one adjustable joint;
   a source configured to emit radiation during an imaging procedure;
   a detector configured to receive attenuated radiation from the source during the imaging procedure, at least one of the source and the detector movable relative to the other by the at least one adjustable joint of the gantry; and
   an imaging controller operably connected to at least the gantry and to the adjustable joint, wherein the gantry controller receives patient information and an imaging system input, the imaging controller determines an imaging geometry based upon the patient information and the imaging system input and operates the gantry and the at least one adjustable joint to adjust a relative position of at least one of the x-ray source and x-ray detector according to the imaging geometry.
2. The system of clause 1 further comprising a movable subject support operably connected to the imaging controller, wherein the system geometry further comprises a position of the movable subject support and the imaging controller operates the movable subject support to the position for the imaging procedure.
3. The system of any preceding clause, wherein the source is a dual-energy x-ray emitter, and the detector is an x-ray detector and the imaging procedure is a dual-energy x-ray absorptiometry (DEXA) procedure.
4. The system of any preceding clause, wherein the x-ray emitter is movably secured to the gantry by an emitter joint and the x-ray detector is movably secured to the gantry by a detector joint, and wherein the emitter joint and the detector joint are both operable by the imaging controller to adjust a source to image receptor distance (SID).
5. The system of any preceding clause, further comprising a collimator positioned relative to the x-ray emitter to produce a fan beam of x-rays.
6. The system of any preceding clause, wherein the patient information is a patient girth.
7. The system of any preceding clause, wherein the patient information is a patient contour relative to the movable subject support.
8. The system of any preceding clause, wherein the patient contour is obtained from a recall image of the patient.
9. The system of any preceding clause, wherein the imaging controller operates to move at least one of the gantry and the movable subject support according to a scanning pattern comprising a plurality of transverse scans, wherein in the imaging geometry at least one of a source-object distance (SOD) and an object-image detector distance (OID) for each transverse scan is adjusted based upon the patient contour.
10. The system of any preceding clause, wherein the imaging system input is an increase in detected x-ray flux and the imaging geometry comprise a reduction in an object-image detector distance.
11. The system of any preceding clause, wherein the imaging system input is a magnification and the imaging geometry comprise a decrease in a source-object distance (SOD).
12. A method of densitometry, the method comprising:
   obtaining patient information of a patient;
   providing an imaging system comprising a movable patient support, an x-ray source movably connected to a movable gantry, and an x-ray detector movably connected to the movable gantry, wherein the patient support, the x-ray source, and the x-ray detector are movable relative to the others;
   determining an imaging geometry from the patient information, wherein the imaging geometry comprises a source-image receptor distance (SID), a source-object distance (SOD), and an object-image receptor distance (OID).
   operating the x-ray source, the x-ray detector and the patient support according to the imaging geometry;
   operating the x-ray source and the x-ray detector to acquire x-ray absorption data; and
   determining a density of an anatomical portion of the patient.
13. The method of any preceding clause, further comprising:
   receiving an imaging system input and wherein the imaging geometry is further determined from the imaging system input.
14. The method of any preceding clause, wherein the imaging system input is an increase in detected x-ray flux and the determined imaging geometry comprises a reduction in the OID.
15. The method of any preceding clause, wherein the imaging system input is a magnification and the imaging geometry comprises a decrease in the SOD.
16. The method of any preceding clause, wherein the patient information is a contour of the patient and further comprising:
   determining a scanning pattern comprising the imaging geometry; and
   executing the scanning pattern by moving at least one of the gantry and the patient support to acquire the x-ray absorption data.
17. The method of any preceding clause, further comprising:
   acquiring a medical image of the patient; and
   determining a patient contour from the medical image of the patient.
18. The method of any preceding clause, wherein the scanning pattern comprises a plurality of transverse scans wherein at least one of the SOD and the OID is adjusted between transverse scans of the plurality of transverse scans based upon the patient contour.
19. A system for densitometry, the system comprising:
   a gantry movable relative to a patient;
   a movable patient support configured to support the patient;
   an x-ray source movably coupled to the gantry and operable to emit radiation during a densitometry procedure;
   a collimator arranged relative to the x-ray source to collimate the emitted radiation to form a fan beam;
   an x-ray detector movably coupled to the gantry and operable to receive attenuated radiation from the x-ray source during the densitometry procedure; and
   an imaging controller that receives patient data and imaging system information and determines a scanning pattern comprising a plurality of transverse scans of the x-ray source and the x-ray detector, the scanning pattern further comprising at least one imaging geometry comprising at least one of a source-object distance (SOD) or an object-image detector distance (OID) determined from the patient data and the imaging system information.
20. The system of any preceding clause, wherein the x-ray source is a dual-energy x-ray emitter and the densitometry procedure is a dual-energy x-ray absorptiometry (DEXA) procedure.

## Claims

1. A system (10) for medical imaging:
a gantry (12) movable relative to a subject, the gantry comprising at least one adjustable joint (50,54);
a source (14) configured to emit radiation during an imaging procedure;
a detector (18) configured to receive attenuated radiation from the source (14) during the imaging procedure, at least one of the source and the detector movable relative to the other by the at least one adjustable joint of the gantry (12); and
an imaging controller (28) operably connected to at least the gantry (12) and to the adjustable joint (50,54), wherein the gantry controller (28) receives patient information and an imaging system input, the imaging controller determines an imaging geometry based upon the patient information and the imaging system input and operates the gantry and the at least one adjustable joint (50,54) to adjust a relative position of at least one of the x-ray source (14) and x-ray detector (18) according to the imaging geometry.

2. The system (10) of claim 1 further comprising a movable subject support operably connected to the imaging controller (28), wherein the system geometry further comprises a position of the movable subject support and the imaging controller (28) operates the movable subject support to the position for the imaging procedure.

3. The system (10) of any preceding claim, wherein the source (14) is a dual-energy x-ray emitter, and the detector (18) is an x-ray detector and the imaging procedure is a dual-energy x-ray absorptiometry (DEXA) procedure.

4. The system (10) of any preceding claim, wherein the x-ray emitter is movably secured to the gantry (12) by an emitter joint and the x-ray detector (18) is movably secured to the gantry by a detector joint, and wherein the emitter joint and the detector joint are both operable by the imaging controller to adjust a source to image receptor distance (SID).

5. The system (10) of any preceding claim, further comprising a collimator (25) positioned relative to the x-ray emitter to produce a fan beam of x-rays.

6. The system (10) of any preceding claim, wherein the patient information is a patient girth.

7. The system (10) of any preceding claim, wherein the patient information is a patient contour relative to the movable subject support.

8. The system (10) of claim 7 wherein the patient contour is obtained from a recall image of the patient.

9. The system (10) of any preceding claim, wherein the imaging controller (28) operates to move at least one of the gantry (12) and the movable subject support according to a scanning pattern comprising a plurality of transverse scans, wherein in the imaging geometry at least one of a source-object distance (SOD) and an object-image detector distance (OID) for each transverse scan is adjusted based upon the patient contour.

10. The system (10) of any preceding claim, wherein the imaging system input is an increase in detected x-ray flux and the imaging geometry comprise a reduction in an object-image detector distance.

11. The system (10) of any preceding claim, wherein the imaging system input is a magnification and the imaging geometry comprise a decrease in a source-object distance (SOD).

12. A method of densitometry, the method comprising:
obtaining patient information of a patient;
providing an imaging system (10) comprising a movable patient support, an x-ray source (14) movably connected to a movable gantry (12), and an x-ray detector (18) movably connected to the movable gantry, wherein the patient support, the x-ray source, and the x-ray detector are movable relative to the others;
determining an imaging geometry from the patient information, wherein the imaging geometry comprises a source-image receptor distance (SID), a source-object distance (SOD), and an object-image receptor distance (OID).
operating the x-ray source (14), the x-ray detector (18) and the patient support according to the imaging geometry;
operating the x-ray source (14) and the x-ray detector (18) to acquire x-ray absorption data; and
determining a density of an anatomical portion of the patient.

13. The method of claim 12, further comprising:
receiving an imaging system input and wherein the imaging geometry is further determined from the imaging system input.

14. The method of claim 13, wherein the imaging system input is an increase in detected x-ray flux and the determined imaging geometry comprises a reduction in the OID.

15. The method of claim 13 or claim 14, wherein the imaging system input is a magnification and the imaging geometry comprises a decrease in the SOD.
